# EUROPEAN PATENT SPECIFICATION

(11) **EP 2 836 123 B1**
(45) Date of publication and mention of the grant of the patent: **10.05.2017**
(21) Application number: 13718017.0
(22) Date of filing: 09.04.2013
(51) Int. Cl.: A61B 5/15, A61B 5/151

(54) **PUSH-TO-CHARGE LANCING DEVICE**
PUSH-TO-CHARGE-LANZETTE
DISPOSITIF DE PERCÉE À POUSSÉE DE CHARGE

(30) Priority: 09.04.2012 US 201261621830 P
(43) Date of publication of application: 18.02.2015
(73) Proprietor: Facet Technologies, LLC, Kennesaw, GA 30144 (US)
(72) Inventor: TRISSEL, John, A., Canton, GA 30115 (US)
(74) Representative: Maschio, Antonio
(86) International application number: PCT/US2013/035746
(87) International publication number: WO 2013/155052

(56) References cited:
- EP-A1- 1 219 242
- WO-A1-2008/098046
- WO-A1-2010/019741
- WO-A1-2010/128701
- DE-A1-102010 004 370

## Description

### Cross-Reference to Related Application

This application claims the priority benefit of U.S. Provisional Patent Application Serial No. 61/621,830 filed April 9, 2012, the entirety of which is hereby incorporated herein by reference.

### Technical Field

The present invention relates generally to the field of medical devices, and more particularly to a lancing device for blood sampling and testing, and an incorporated mechanism for charging the lancet drive mechanism by pushing an exposed portion of the mechanism into the lancing device.

### Background

Lancing devices are utilized for penetrating the skin of a human or animal subject at a lancing site to obtain a sample of blood or other body fluid for medical testing, as in blood-typing or blood-glucose testing. Known lancing devices commonly include a housing containing a drive mechanism, a charging mechanism for energizing the spring or other drive means of the drive mechanism, and a release mechanism for releasing the drive mechanism upon actuation. U.S. Patent App. Serial No. 13/005,181 (Pub. No. US 2011/0196261) and U.S. Patent App. Serial No. 12/641,674 (Pub. No. US 2010/0160942) show example lancing devices.

A lancet is typically propelled by the drive mechanism from a retracted position within the housing to an extended position wherein a sharp tip portion of the lancet projects from the housing to prick the subject's skin at a desired lancing site. Many known lancing devices commonly use a drive mechanism that is charged or energized by pulling the drive mechanism to a retracted position, generally away from the body of the lancing device, resulting in the user having to perform the charging procedure by actuating or pulling the charging mechanism away from the body of the lancing device. Charging the drive mechanism by pulling the charging mechanism away from the body of the lancing device can present challenges to users with reduced manual dexterity, and may require the subject or user to use two hands to hold the device body and pull the handle until the device is charged and ready to activate. EP 1219242 discloses a body fluid sampler including first and second plunger rods connected via a spring, a latch mechanism and a release mechanism. The latch mechanism comprises first and second engagement members supported at both ends, for restricting movement of the first and second plunger rod. A release mechanism is provided for releasing the latched state of the first and second plunger rod. WO 2010/019741 discloses a lancing device comprising: a lancet; a lancet holder receiving the lancet and configured to move axially in a housing between a retracted position, an extending position and a cocked position; a cocking mechanism; and a trigger. The trigger has a guide structure for slidable receipt of the cocking mechanism. Upon actuation of the trigger, the lancet holder is released from the cocked position to the extended position. DE 102010004370 discloses a lancing device comprising a lancet holder that is linearly displaceable within a housing. In use, an operating knob is actuated such that projections on a latching hook come into contact with projections on a trip slider, pushing it upwards and compressing a spring. As the operating knob is further actuated, the trip slider comes into contact with abutments on the housing, causing the latching hooks to disengage from the lancet holder. The charging and release of the lancet holder are effected by a single downward actuation of the operating knob. WO 2010/128701 discloses a lancing device comprising a lancet mounting device having an elastic body and being slidably received within a housing. To charge the lancing device, the lancet mounting device is retracted so as to compress a compression spring and engage the elastic body behind a load button extending from the housing. To release the lancet mounting device, a trigger is actuated so as to release the elastic body from the load button. WO 2008/098046 discloses a lancet device comprising a housing for slidably receiving a holding member and a push-button. The holding member comprises a deflecting member that engages with a shoulder of the housing when the holding member is in a charged state. The user fires a trigger to move the deflecting member out of engagement with the shoulder, releasing the holding member in a forward direction.

Needs exist for improved systems and methods for charging of lancing devices. It is to the provision of improved lancing devices and methods of operation and use thereof that the present invention is primarily directed.

### Summary

In example embodiments, the present invention provides a lancing device having improved drive and charging features. A charging mechanism is provided for actuating into a lancet carrier of the lancing device for charging a drive spring. The drive spring is partially housed within a slot of the lancet carrier wherein a piston is translatably mounted to receive a portion of the charging mechanism upon actuation, further charging the drive spring. A release mechanism is provided for actuating the lancet carrier. Additional example embodiments of the present invention provide improved methods of use of lancing devices.

In one aspect, the present invention relates to a lancing device for completing a lancing stroke. The lancing device includes a lancet carrier with a distal end and a proximal end. The lancing device also includes a drive mechanism to drive the lancet carrier through the lancing stroke. The lancing device also includes a charge mechanism to charge the drive mechanism. The charge mechanism is configured to apply a charging force onto the drive mechanism by pushing the charge mechanism along a common direction with the lancing stroke. The lancing device also includes a piston with a proximal end and a distal end. The piston distal end engages the drive mechanism and the piston proximal end engages the charge mechanism. The lancing device also includes at least one detent. The detent includes a resiliently-flexible cantilevered arm having an attached proximal end and a distal free end. The cantilevered arm comprises a ramped surface at the distal free end. The piston proximal end releasably engages the at least one detent when the drive mechanism is charged by the charge mechanism.

The lancing device may include a housing with a proximal end and a distal end and a hollow core. The proximal end may include an aperture. The lancet carrier may be translatably supported within the housing. The drive mechanism may be supported with respect to the lancet carrier. The drive mechanism may include a relaxed state and a charged state. The charge mechanism may be translatably inserted through the housing proximal end aperture.

In another aspect, the invention relates to a method for charging a drive mechanism in a lancing device. The method includes engaging a charge mechanism with respect to a drive mechanism that is translatably supported within a lancet carrier. The lancet carrier includes a proximal end and a distal end, and the drive mechanism includes a relaxed state and a charged state. The method also includes pushing the charge mechanism towards the lancet carrier distal end and releasably engaging the drive mechanism with respect to a detent on the lancing device when the drive mechanism is in the charged state.

These and other aspects, features and advantages of the invention will be understood with reference to the drawing figures and detailed description herein, and will be realized by means of the various elements and combinations particularly pointed out in the appended claims. It is to be understood that both the foregoing general description and the following brief description of the drawings and detailed description of the invention are exemplary and explanatory of preferred embodiments of the invention, and are not restrictive of the invention, as claimed.

### Brief Description of the Drawings

**FIGURE 1A** is an underneath perspective view of a lancing device according to an example embodiment of the present invention.
**FIGURE 1B** is a top perspective view of the lancing device of **FIGURE 1A**.
**FIGURES 2A** and **2B** show internal views, with the housing removed and in a neutral state, of the lancing device of **FIGURES 1A** and **1B**.
**FIGURE 3** is an exploded assembly view of the lancing device of **FIGURES 1A** and **1B**.
**FIGURE 4A** is an isolated top perspective view of the housing portion of the lancing device of **FIGURE 3**.
**FIGURE 4B** is an isolated top perspective view of a lancet carrier portion of the lancet carrier of **FIGURE 3**.
**FIGURE 5** is an isolated perspective view of the piston-receiving portion of the lancet carrier of **FIGURE 4B**.
**FIGURE 6** is an isolated perspective view of the lancet-carrier portion of the lancet carrier of **FIGURE 4B**.
**FIGURE 7** is a front view of the lancet-carrier portion of the lancet carrier in **FIGURE 6**, as viewed along line A.
**FIGURE 8** is a rear view of the lancet-carrier portion of the lancet carrier in **FIGURE 6**, as viewed along line B.
**FIGURES 9A-9E** are cross-sectional views of the lancing device of **FIGURES 1A** and **1B**, showing the sequential operation moving between a neutral state, a charging state, a charged state, and a fully extended state, and returning to the neutral state.
**FIGURES 10A-10E** are cross-sectional views of a lancing device according to another example embodiment of the invention, showing the sequential operation moving between a neutral state, a charging state, a charged state, and a fully extended state, and returning to the neutral state.

### Detailed Description of Example Embodiments

The present invention may be understood more readily by reference to the following detailed description of the invention taken in connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention.

Also, as used in the specification including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

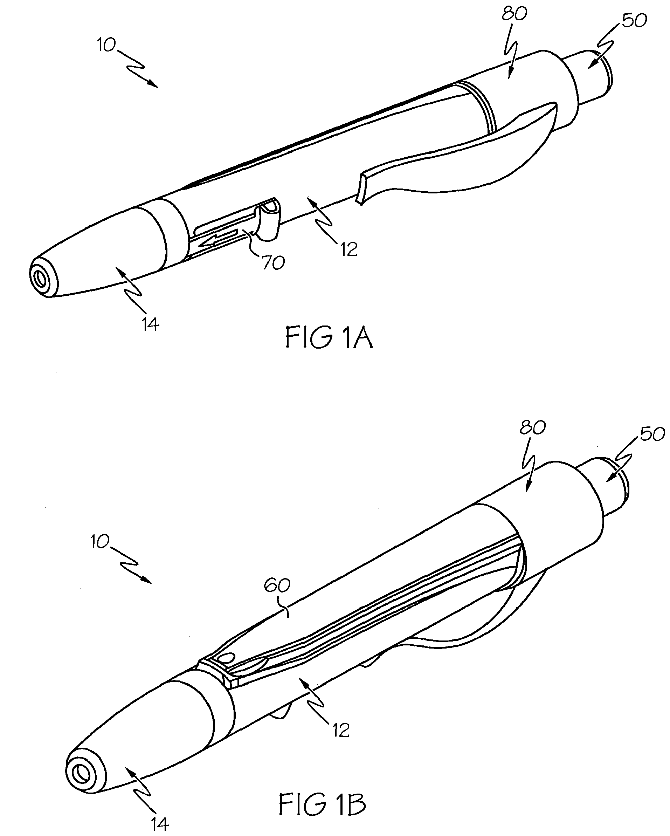

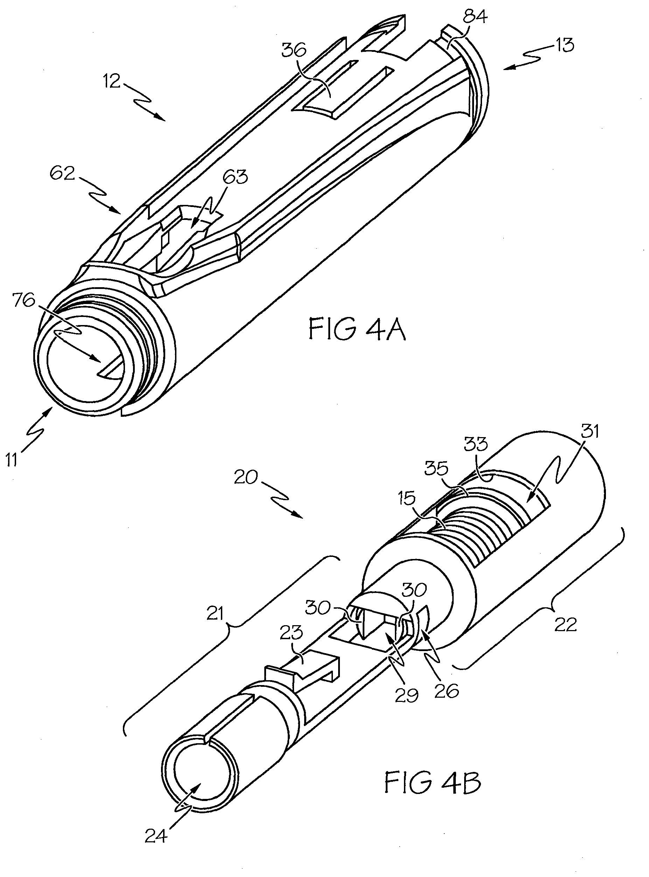

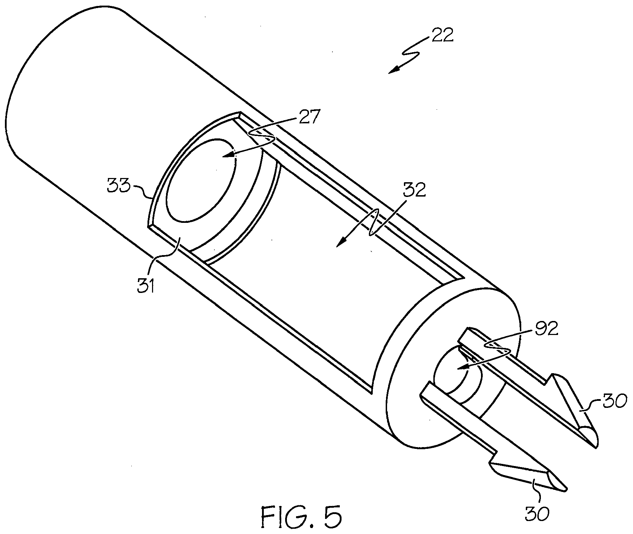

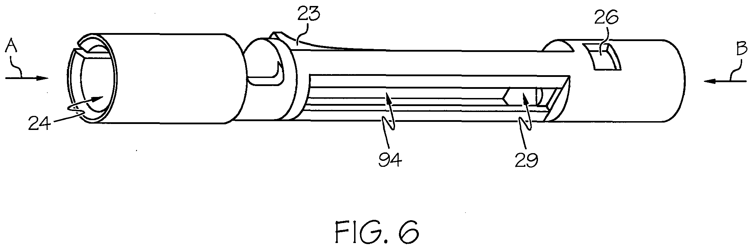

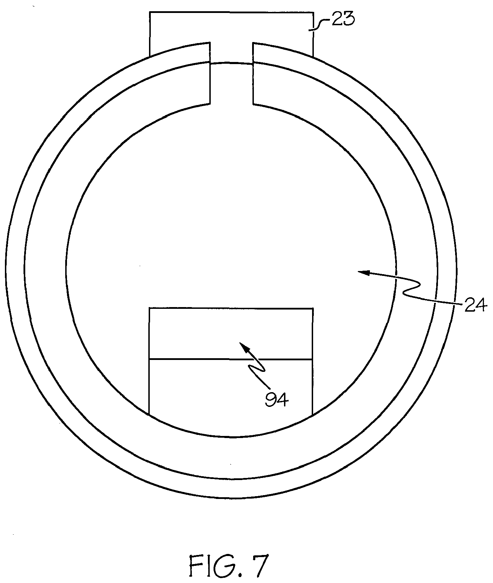

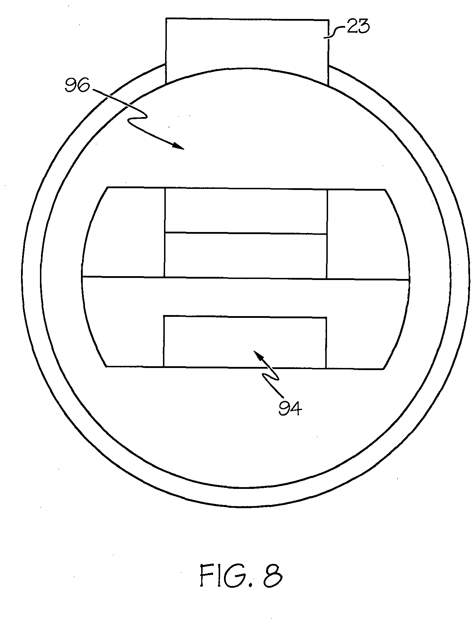

With reference now to the drawing figures, wherein like reference numbers represent corresponding parts throughout the several views, **Figures 1 - 9E** show a lancing device 10 according to an example embodiment of the invention. The lancing device 10 generally includes a housing 12 and an end cap 14 removably secured to the housing, for example through corresponding threaded surfaces or friction fit. The housing 12 at least partially encloses a lancing-stroke drive mechanism that includes a drive spring 15 for driving a lancet carrier 20 along a lancing stroke, a drive piston 34 for applying an anti-bias compressive force to the drive spring and a return spring 16 for returning the lancet carrier to a neutral position. A charging plunger 50 is provided for charging the lancing-stroke mechanism by actuating the drive piston 34 in translation against the bias of the drive spring 15.

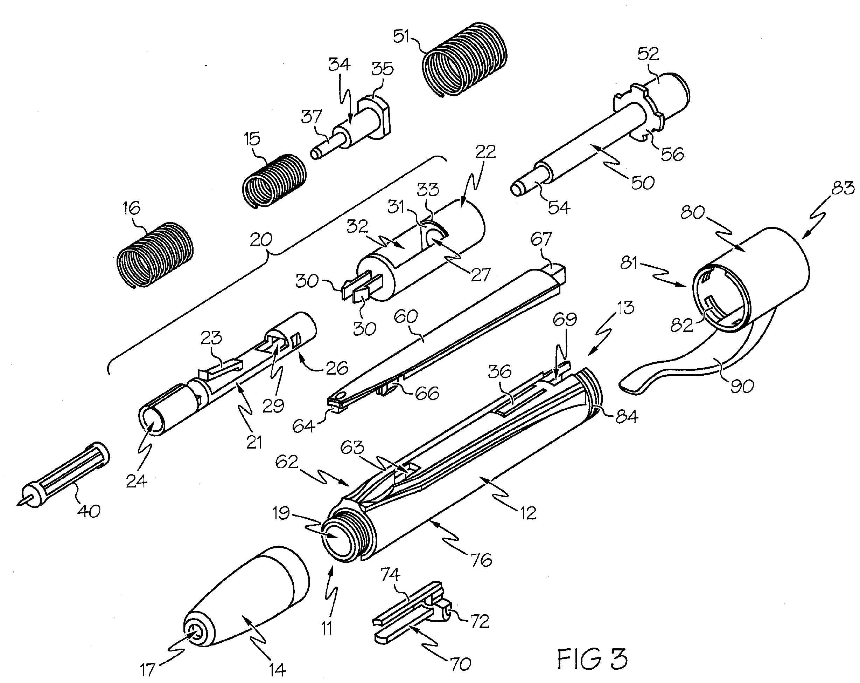

**Figure 3** shows an exploded assembly view of the lancing device 10. As depicted, the drive piston 34 applies an anti-bias compressive force on the drive spring 15 along a common axial direction with the lancing stroke. The piston 34 is traversed distally of one or more interengaging detents or catch features 36 extending within the housing 12, to hold the drive spring 15 in a charged state before entering the lancing stroke. The detent or catch feature 36 includes a resiliently-flexible cantilevered arm having a ramped surface or shaped detent, at the distal free end, to releasably engage and retain the drive piston base 35 flange.

The housing 12 generally includes an elongate member having a front distal end 11 and a rear proximal end 13 generally opposite thereto. The front distal end 11 includes a threaded outer surface and a longitudinally-oriented aperture 19. Opposing vertically-oriented apertures 62 and 76 are provided near the housing front end 11 to receive the release mechanism 60, as described above, and an ejector 70 for ejecting a used lancet 40.

The lancet carrier 20 generally includes two members, a lancet-carrying member 21 and the piston-carrying member 22. The lancet-carrying member 21 carries the lancet 40 along a lancing stroke and selectively engages the housing 12 to prevent relative movement therein. The lancet-carrying member 21 has a distal end with a receiver cavity 24 for receiving and holding a lancet 40 throughout the lancing procedure. The lancet-carrying member 21 includes a release finger 23 integrally formed with or attached thereon. Preferably, the release finger 23 has a cantilevered shape and includes a distal free end having a sloped surface or feature to catch or run within the interior of the housing 12. The lancet-carrying member 21 also includes a proximal end with a receiver feature 26.

The piston-carrying member 22 has a distal end and a proximal end that includes a cylindrical body with a distal end face 31 and a proximal end face. The distal end of the piston-carrying member 22 includes an aperture 92. A hollow elongated channel 27 extends through the proximal cylindrical body between the distal face 31 and the proximal face. The distal end aperture 92 is axially aligned with the hollow elongated channel 27.

A slot chamber 32 extends between the distal end of the piston-carrying member 22 and the distal face 31 of the proximal cylindrical body of the piston-carrying member. The slot chamber 32 can have a generally circumferential geometry with opposing apertures along the length of each side. The overhang flange 33 extends from the proximal end body of the piston-carrying member 22 over the slot chamber 32, forming a recession area underneath the overhang flange. The piston-carrying member 22 functions to translatably support or receive the drive piston 34 within the slot chamber 32.

The piston-carrying member 22 distal end secures to the lancet-carrying member 21, preferably with one or more cooperating connection features. For example, the lancet-carrying member receiver mechanism 26 can include a pair of opposing apertures providing access to a hollow internal core 29. A pair of resilient probes 30 with outwardly-oriented barbs can protrude from the distal end of the piston-carrying member 22. The resilient probe barbs 30 insert into the lancet-carrying core through the proximal end and secure through the opposing apertures 26.

As depicted, the drive piston 34 has a base support 35 and a probe nose 37 extending distally away from the base support. The base support 35 can have a generally-circumferential flange. Optionally, the base support 35 can have a hollow bore interior (not shown) for receiving the charging plunger 50. The drive piston 34 is naturally biased by the drive spring 15 applying a force on the flange of the base support 35 towards a face surface 31 recessed under an overhang flange 33 at the proximal end of the distal slot chamber 32. The distal end of the piston probe nose 37 slidably inserts through the aperture 92 in the distal end of the piston-carrying member 22.

The slot chamber 32 translatably receives the drive piston 34 and the drive spring 15 that is secured around the piston nose probe 37. The drive spring 15 is retained between a front distal end of the slot chamber 32 and the piston base flange 35. The bias of the drive spring 15 urges the base flange 35 to be positioned or nest against the recessed face surface 31 underneath the overhang flange 33. Preferably, the piston base 35 flange and the recessed region under the overhang flange 31 have a substantially similar contour.

When the piston base 35 bore and the elongated channel aperture 27 are axially aligned, the charging plunger 50 can extend through the elongated hollow channel and insert into the piston base bore. The charging plunger 50 generally includes an elongated body with a button 52 and the elongated probe nose 54. The distal end of the elongated probe nose 54 inserts into the proximal end aperture of the hollow elongated channel 27 and translatably exits the distal end aperture of the hollow elongated channel. Optionally, an internal bore (not shown) extending from the flange on the piston base 35 can align with the distal front aperture of the elongated hollow channel 27 to receive the distal end of the elongated probe nose. The piston probe nose 37 then inserts through distal aperture 92, so that the piston is translatably supported by the aperture 92 and the distal end 54 of the charging plunger 50.

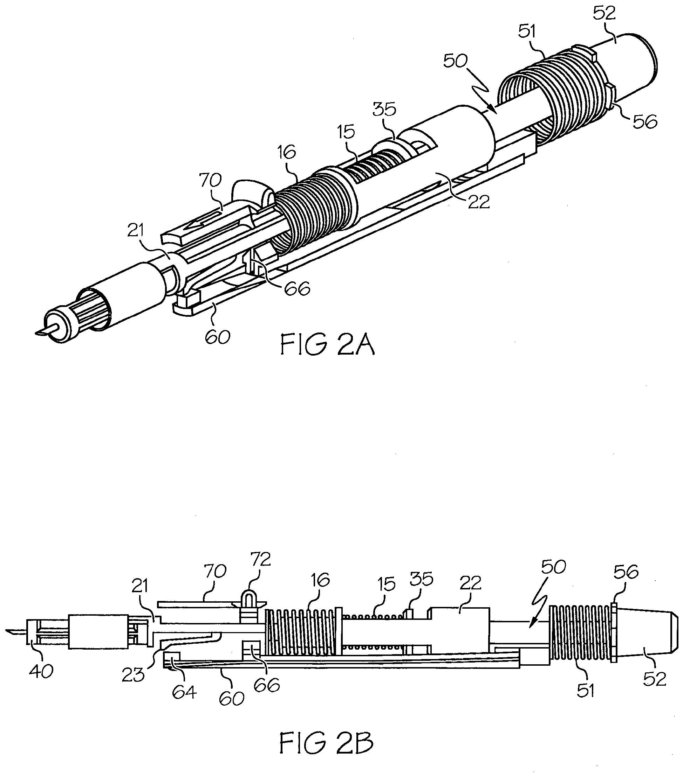

Application of a distally-directed force to the button 52 by a user forces the drive piston 34 in translation against the bias of the drive spring 15 secured around the piston. This distally-directed translation of the piston 34 causes the drive spring 15 to compress between the piston base 35 flange and the distal end face of the slot chamber 32. The return spring 16 is secured around the lancet-carrying member 21 between the front face of the piston-carrying member 22 distal end and a combination of the release mechanism fastener 66 and the ejector arm 74, as best shown in **Figures 2A & 2B**.

The charging plunger 50 has a retention guide 56 that is integrally formed between the elongated probe nose 54 and the button 52 extends generally perpendicular to the axis of the elongated plunger body and comprises four radially offset arms or pedals that extend beyond the circumferential diameter of the button.

A release mechanism or button 60 is provided for removing a release finger 23 on the lancet carrier 20 from engagement with the distal end of a trigger catch 62 on the housing 12. When the release finger 23 is disengaged from the distal end of the trigger catch 62, the lancet carrier 20 is naturally driven, by the bias of the drive spring 15 against the drive piston 34, towards an aperture 17 in the distal end of the end cap 14. The sharp tip of a lancet 40, being carried by the driven lancet carrier 20, projects through the aperture 17 to be used on a subject's skin at a lancing site.

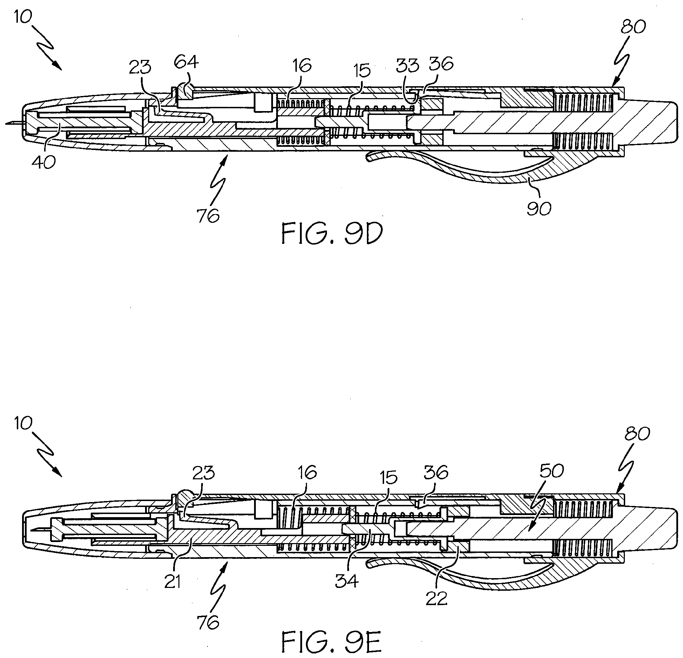

When the lancet carrier 20 is driven forward by the drive spring 15, the recessed region under the overhang flange 33 receives the piston base 35 and the overhang flange then engages the detents or catch feature 36. Upon the lancet carrier 20 being driven toward the advanced position within the housing 12 by the drive spring 15, the overhang flange 33 engages the catch feature 36 and flexes the cantilevered arm free end upwardly to slide along the outer surface of the piston-receiving member 22 proximal end, as best shown in **Figure 9D**. This engagement disengages the detents or catch features 36 from the piston base 35 flange and then upward onto the exterior surface of the proximal region of a piston-receiving member 22 of the lancet carrier 20.

As a consequence of the natural bias of the drive spring 15, the return spring 16 is compressed by an anti-bias force applied by the piston-receiving member 22. After the lancet carrier 20 is driven distally towards the end cap aperture 17, the return spring 16 then naturally biases the lancet carrier away from the aperture towards the neutral position, as best shown in **Figure 9E**. With the piston base 35 within the recession under the overhang flange 33, the detent or catch feature 36 then can slide over the overhang flange to allow the lancet carrier 20 to return to the neutral state.

When the return spring 16 pushes the drive piston 34 to return to a neutral state within the recession underneath the overhang flange 33, the lancet carrier returns to a first position at a position proximal to the catch feature 36, as best shown in **Figure 9E**.

The release mechanism 60 has a generally elongated body with a deformably-resilient distal end region. The distal end region includes a button 64 and a fastener 66, for example a pair of resiliently-flexible outwardly-facing barbs, extending from the underside at a position proximal from the distal end. The proximal end of the release mechanism 60 includes an insert 67 extending from the underside of the proximal end.

In use, the release mechanism 60 is secured onto the housing 12. The housing catch 62 can be an aperture with a narrowed proximal region 63. The release mechanism fastener 66 snaps into the narrowed proximal region 63 of the distal aperture 62 and the proximal insert 67 fits within the proximal cutout 69. When the release mechanism 60 is secured to the housing 12, the distal button 64 inserts through the distal catch aperture 62 and engages the sloped distal end of the cantilevered arm 23 on the lancet-carrying member 21. The deformably-resilient distal end of the release mechanism 60 can be depressed so that the button 64 applies a downward force to the sloped distal end of the cantilevered finger 23 to disengage the cantilevered finger from the catch aperture 62.

The housing 12 also has a cutout 69 positioned at the proximal end of the housing. The insert 67 of the release mechanism 60 can engage the proximal end face of the piston-carrying member 22 to prevent the lancet carrier 20 from being pushed in a proximal or reversed direction towards the proximal end 13 of the housing 12.

The ejector 70 generally includes a main elongate body and a handle 72 that protrudes away from the main elongate body. A cantilevered arm 74 is generally laterally offset from, and parallel to, the elongate body and extends the length of the elongate body. The ejector 70 preferably has a uniform construction, for example through injection molding. An elongated channel 94, with proximal and distal apertures, extends along the underside of the lancet-receiving member 21 between the receiver cavity 24 and a midpoint along the lancet-receiving member body. The channel 94 receives the arm 74 and allows the arm to translatably move proximally and distally with respect to the lancet-receiving member 21. In use, a distally-directed user-applied force applied to the ejector handle 72 translates the arm 74 along the elongated channel 94 in the receiver cavity 25, and through the channel distal aperture, so that the arm applies a distally-oriented force against a used lancet 40 in the receiver cavity to eject the lancet from the receiver cavity. When the described ejection process is performed, the end cap 14 is preferably removed from the distal end 11 of the housing 12.

A collar 80 can secure around the plunger button 52. The collar 80 generally includes a hollow circumferential interior extending between a distal open end 81 and a proximal open end with an inwardly-directed lip (not shown). Preferably, the distal open end 81 has one or more generally circumferentially-shaped detents or catching features 82 positioned to catch or engage at least one corresponding detent or catching feature 84 positioned around the proximal end 13 of the housing 12. The distal open end 81 fits over the button 52 and the retention guide 56 arms snap over the detents or catching features 82. Then, the button 52 extends outwardly from the collar 80 through the proximal end 83 aperture (not shown). The retention guide 56 arms engage the inner surface of the proximal end 83 lip (not shown) to prevent the plunger 50 from exiting through the proximal end 83 aperture (not shown) of the collar. Optionally, a clip or arm member 90 may be integrally formed with or attached to the collar 80 for coupling the lancing device 10, for example by engaging the clip with a pocket or an article of clothing.

As best shown in **Figures 2A** and **2B**, a biasing spring 51 is retained around the probe nose 54 on the elongated body of the charging plunger 50 between the retention guide 56 and the housing proximal end 13. The biasing spring 51 biases the charging plunger 50 away from the housing proximal end 13.

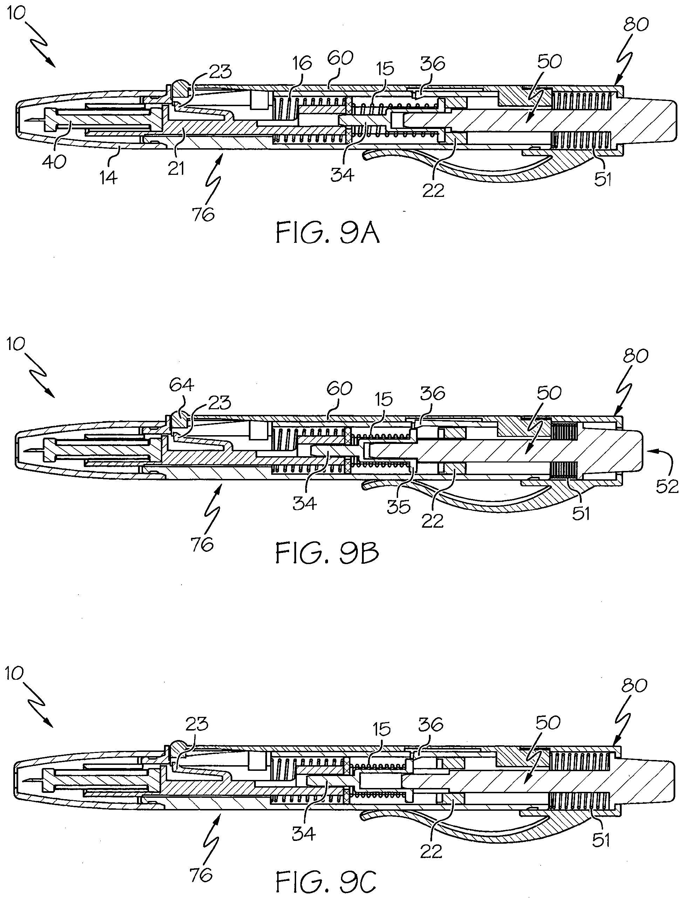

In the sequence of operation, the lancet carrier 20 translates from a neutral or first position, as depicted in **Figure 9A**, to an advanced position, depicted in **Figure 9D**, and back to a neutral position shown in **Figure 9E**. In the neutral position shown in **Figure 9A**, the release finger 23 of the lancet carrier 20 engages the trigger catch 62 of the housing 12, prohibiting the lancet carrier 20 from moving towards the distal end 11 of the housing and end cap aperture 17. The drive spring 15 biases against the base 35 flange of the drive piston 34 towards the recessed face surface 31 underneath the overhang flange 32. When the button 52 of the charging plunger 50 is pressed into the device, the probe nose 54 actuates forward to engage the base 35 of the drive piston 34, further actuating the base 35 distally beyond the resilient cantilevered catch feature 36 to hold the drive piston 34 in a charged state shown in **Figure 9B**. In the charged state, the drive spring 15 is compressed against bias between the piston base 35 and the distal end of the slot chamber 32. Once the drive mechanism is charged, the biasing spring 51 biases the charging plunger 50 back to a neutral position shown in **Figure 9C**.

When the lancing device is fired by depression of the distal end of the release mechanism 60, as previously described, the bias of the drive spring 15 against the base 35 drives the lancet carrier 20 in a distal direction, shown in **Figure 9D**, into an advanced position for penetrating the subject's skin at the lancing site. During this lancet stroke, the bias of the drive spring 15 forces lancet carrier 20 in a distal direction to receive the drive piston 34 into the recessed face surface 31 under the overhang flange 33. In the depicted embodiment, the forward extent of travel of the lancet carrier is limited by contact of the lancet with the inner face around the aperture 17 of the cap 14.

Because the bias of the drive spring 15 forces the entire lancet carrier 20 in a distal direction, the overhang flange 33 moves to engage and outwardly-flex the catch feature 36 onto the outer surface of the piston-carrying member proximal end body. As a consequence of this distal movement by the lancet carrier 22, the return spring 16 is also compressed against bias. When the drive spring 15 has fully biased and the catch feature 36 is slidably engaged with the outer surface of the piston-carrying member proximal body, the natural bias of the return spring 16 forces the lancet carrier 20 proximally back to a neutral position to complete the lancing stroke, shown in **Figure 9E**.

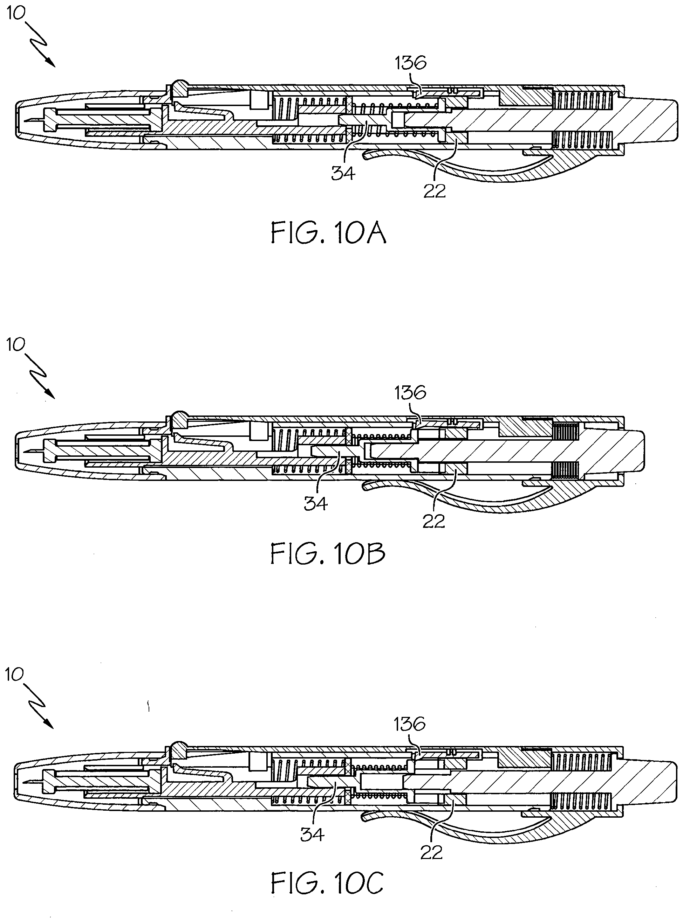

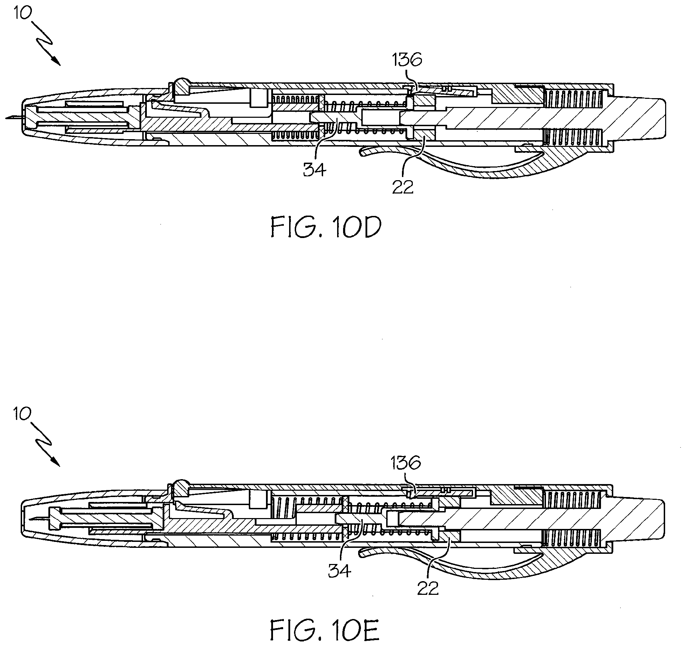

In further example embodiments, the catch feature pivotally mounts to the housing for engaging the head of the drive piston when actuated by the charging mechanism. For example, as depicted in **Figures 10A-10E**, a pivotal catch feature 136, having a substantially similar shape as the catch feature 36, pivotally mounts to the housing near the proximal end 13. The operation of the pivotal catch feature 136 throughout the sequencing of the lancing device 10 is generally similar to the above-described catch feature 36, and similarly retains the drive piston 34 in a charged state until a portion of the lancet carrier 20 removes the catch feature from the drive piston, as best shown in **Figure 10D**. Preferably, the proximal end of the piston-carrying member 22 is generally short in length to allow a portion of the pivotal catch feature 136 to pivot into the housing 12 when the flange overhang 33 removes the catch feature from the drive piston 34. Additionally, as depicted in **Figures 10B-10C**, the position of the proximal portion of the lancet carrier 20 in the first position biases or prohibits the pivotal catch 136 from releasing and pivoting therein, ensuring that the catch remains engaged and latched with the piston 34 until the lancet carrier is advanced. When the lancet carrier 20 is in a forward or extended position the pivotal catch 136 is free to rotate and release the drive piston 34. In additional example embodiments, the pivotal catch feature 136 can include one or more biasing members or springs to bias the catch feature 136 to partially extend beyond the internal surface of the housing 12.

The system and method of example forms of the invention enable a user to charge the drive mechanism of a lancing device by pushing or pressing an actuator portion in an advancing or forward direction to energize the drive spring, in the familiar manner of operating a retractable ball-point pen. Upon actuation of the release mechanism, the drive mechanism propels the lancet along an advancing portion of a lancing stroke, also in the advancing or forward direction. In example embodiments, the lancet is generally coaxially aligned with the actuator portion of the drive mechanism.

While the invention has been described with reference to preferred and example embodiments, it will be understood by those skilled in the art that a variety of modifications, additions and deletions are within the scope of the invention, as defined by the following claims.

## Claims

1. A lancing device (10) for completing a lancing stroke, the lancing device (10) comprising:
a lancet carrier (20) comprising a distal end and a proximal end;
a drive mechanism (15, 16, 34) to drive the lancet carrier (20) through the lancing stroke;
a charge mechanism to charge the drive mechanism (15, 16, 34), the charge mechanism configured to apply a charging force onto the drive mechanism (15, 16, 34) by pushing the charge mechanism along a common direction with the lancing stroke;
a piston (34) with a proximal end and a distal end, wherein the piston distal end engages the drive mechanism (15, 16, 34) and the piston proximal end engages the charge mechanism; and
at least one detent (36), **characterized in that** the detent (36) includes a resiliently-flexible cantilevered arm having an attached proximal end and a distal free end, wherein the cantilevered arm comprises a ramped surface at the distal free end, and wherein the piston proximal end releasably engages the at least one detent (36) when the drive mechanism (15, 16, 34) is charged by the charge mechanism.

2. The lancing device (10) of Claim 1, wherein the drive mechanism (15, 16, 34) comprises a drive spring (15) that biases in an opposite direction than the lancing stroke.

3. The lancing device (10) of Claim 1, wherein the piston (34) is translationally secured between the lancet carrier distal end and the lancet carrier proximal end.

4. The lancing device (10) of Claim 1, wherein the lancet carrier (20) is configured to disengage with respect to the at least one detent (36) and the drive mechanism (15, 16, 34) during the lancing stroke.

5. The lancing device (10) of Claim 4, wherein the lancet carrier (20) comprises an overhang flange (33) configured to disengage with respect to the at least one detent (36) and the drive mechanism (15, 16, 34).

6. The lancing device (10) of Claim 1, wherein the charge mechanism comprises a plunger (50), the plunger (50) being translationally supported with respect to the lancet carrier (20).

7. The lancing device (10) of claim 1, further comprising a housing (12) comprising a proximal end (13) and a distal end (11) and a hollow core, the proximal end (13) comprising an aperture;
wherein the lancet carrier (20) is translationally supported within the housing (12);
wherein the drive mechanism (15, 16, 34) is supported with respect to the lancet carrier (20), the drive mechanism (15, 16, 34) comprising a relaxed state and a charged state; and
wherein the charge mechanism is translationally inserted through the housing proximal end aperture.

8. The lancing device (10) of Claim 7, wherein the cantilevered arm extends from the housing (12).

9. The lancing device (10) of Claim 8, wherein the lancet carrier (20) comprises a hollow body (32) extending from the lancet carrier proximal end toward the lancet carrier distal end, the lancet carrier hollow body (32) is configured to disengage the ramped surface from the drive mechanism (15, 16, 34).

10. The lancing device of Claim 7, wherein the drive mechanism comprises a drive spring and the piston is translationally supported between the lancet carrier proximal end and the lancet carrier distal end, wherein the piston engages the drive spring.

11. The lancing device (10) of Claim 10, wherein the drive spring (15) is compressed during the drive mechanism charged state.

12. The lancing device (10) of Claim 11, wherein the drive spring (15) is biased oppositely to the charge mechanism push engagement direction.

13. A method for charging a drive mechanism (15, 16, 34) in a lancing device (10) according to claim 1, the method comprising the steps of:
engaging the charge mechanism with respect to the drive mechanism (15, 16, 34) that is translationally supported within the lancet carrier (20), the drive mechanism (15, 16, 34) comprising a relaxed state and a charged state;
pushing the charge mechanism towards the lancet carrier distal end; and
releasably engaging the drive mechanism (15, 16, 34) with respect to the at least one detent (36) on the lancing device when the drive mechanism (15, 16, 34) is in the charged state.

## Patentansprüche

1. Stechhilfe (10) zum Vervollständigen eines Stechhubs, die Stechhilfe (10) umfassend:
einen Lanzettenträger (20), aufweisend ein distales Ende und ein proximales Ende;
einen Antriebsmechanismus (15, 16, 34), um den Lanzettenträger (20) während des Stechhubs anzutreiben;
einen Lademechanismus, um den Antriebsmechanismus (15, 16, 34) zu laden, wobei der Lademechanismus zum Aufbringen einer Ladekraft auf den Antriebsmechanismus (15, 16, 34) durch Schieben des Lademechanismus entlang einer gemeinsamen Richtung mit dem Stechhub ausgebildet ist;
einen Kolben (34) mit einem proximalen Ende und einem distalen Ende, wobei das distale Ende des Kolbens mit dem Antriebsmechanismus (15, 16, 34) im Eingriff steht und das proximale Ende des Kolbens mit dem Lademechanismus im Eingriff steht; und
zumindest eine Feststellvorrichtung (36), **dadurch gekennzeichnet, dass** die Feststellvorrichtung (36) einen elastisch flexiblen freitragenden Arm mit einem befestigten proximalen Ende und einem freien distalen Ende aufweist, wobei der freitragende Arm an dem freien distalen Ende eine geneigte Oberfläche umfasst, und wobei das proximale Ende des Kolbens lösbar mit der zumindest einen Feststellvorrichtung (36) im Eingriff steht, wenn der Antriebsmechanismus (15, 16, 34) von dem Lademechanismus geladen ist.

2. Stechhilfe (10) nach Anspruch 1, wobei der Antriebsmechanismus (15, 16, 34) eine Antriebsfeder (15) umfasst, die in einer entgegengesetzten Richtung wie der Stechhub vorspannt.

3. Stechhilfe (10) nach Anspruch 1, wobei der Kolben (34) translatorisch zwischen dem distalen Ende des Lanzettenträgers und dem proximalen Ende des Lanzettenträgers befestigt ist.

4. Stechhilfe (10) nach Anspruch 1, wobei der Lanzettenträger (20) zum Auskuppeln bezüglich der zumindest einen Feststellvorrichtung (36) und dem Antriebsmechanismus (15, 16, 34) während des Stechhubs ausgebildet ist.

5. Stechhilfe (10) nach Anspruch 4, wobei der Lanzettenträger (20) einen überstehenden Flansch (33) umfasst, der zum Auskuppeln bezüglich der zumindest einen Feststellvorrichtung (36) und dem Antriebsmechanismus (15, 16, 34) ausgebildet ist.

6. Stechhilfe (10) nach Anspruch 1, wobei der Lademechanismus einen Stempel (50) umfasst, wobei der Stempel (50) translatorisch bezüglich dem Lanzettenträger (20) gestützt ist.

7. Stechhilfe (10) nach Anspruch 1, weiterhin umfassend ein Gehäuse (12), das ein proximales Ende (13) und ein distales Ende (11) und einen hohlen Kern aufweist, wobei das proximale Ende (13) eine Öffnung umfasst;
wobei der Lanzettenträger (20) translatorisch in dem Gehäuse (12) gestützt ist;
wobei der Antriebsmechanismus (15, 16, 34) bezüglich des Lanzettenträgers (20) gestützt ist, wobei der Antriebsmechanismus (15, 16, 34) einen entspannten Zustand und einen geladenen Zustand umfasst; und
wobei der Lademechanismus translatorisch durch die Öffnung des proximalen Endes des Gehäuses eingesetzt wird.

8. Stechhilfe (10) nach Anspruch 7, wobei der freitragende Arm sich von dem Gehäuse (12) erstreckt.

9. Stechhilfe (10) nach Anspruch 8, wobei der Lanzettenträger (20) einen hohlen Körper (32) umfasst, der sich von dem proximalen Ende des Lanzettenträgers zu dem distalen Ende des Lanzettenträgers erstreckt, wobei der hohle Körper (32) des Lanzettenträgers zum Lösen der geneigten Oberfläche von dem Antriebsmechanismus (15, 16, 34) ausgebildet ist.

10. Stechhilfe nach Anspruch 7, wobei der Antriebsmechanismus eine Antriebsfeder umfasst und der Kolben translatorisch zwischen dem proximalen Ende des Lanzettenträgers und dem distalen Ende des Lanzettenträgers gestützt ist, wobei der Kolben in die Antriebsfeder eingreift.

11. Stechhilfe (10) nach Anspruch 10, wobei die Antriebsfeder (15) komprimiert ist, während der Antriebsmechanismus in dem geladenen Zustand ist.

12. Stechhilfe (10) nach Anspruch 11, wobei die Antriebsfeder (15) entgegengesetzt zu der Richtung des Schiebeeingriffs des Lademechanismus vorgespannt ist.

13. Verfahren zum Laden eines Antriebsmechanismus (15, 16, 34) in einer Stechhilfe (10) nach Anspruch 1, das Verfahren umfassend die Schritte:
in Eingriff bringen des Lademechanismus mit dem Antriebsmechanismus (15, 16, 34), der translatorisch in dem Lanzettenträger (20) gestützt ist, wobei der Antriebsmechanismus (15, 16, 34) einen entspannten Zustand und einen geladenen Zustand umfasst;
Schieben des Lademechanismus in Richtung des distalen Endes des Lanzettenträgers; und
lösbar in Eingriff bringen des Antriebsmechanismus (15, 16, 34) mit der zumindest einen Feststellvorrichtung (36) an der Stechhilfe, wenn der Antriebsmechanismus (15, 16, 34) in dem geladenen Zustand ist.

## Revendications

1. Dispositif de percée (10) pour réaliser une course de percée, le dispositif de percée (10) comprenant :
un support (20) de lancette comprenant une extrémité distale et une extrémité proximale ;
un mécanisme d'entraînement (15, 16, 34) pour entraîner le support (20) de lancette lors de la course de percée ;
un mécanisme de charge pour charger le mécanisme d'entraînement (15, 16, 34), le mécanisme de charge étant configuré pour appliquer une force de charge au mécanisme d'entraînement (15, 16, 34) en poussant le mécanisme de charge le long d'une direction commune avec la course de percée ;
un piston (34) ayant une extrémité proximale et une extrémité distale, dans lequel l'extrémité distale du piston s'engage avec le mécanisme d'entraînement (15, 16, 34) et l'extrémité proximale du piston s'engage avec le mécanisme de charge ; et
au moins un cran d'arrêt (36), **caractérisé en ce que** le cran d'arrêt (36) inclut un bras en porte-à-faux élastiquement flexible ayant une extrémité proximale attachée et une extrémité distale libre, dans lequel le bras en porte-à-faux comprend une surface inclinée au niveau de l'extrémité distale libre, et dans lequel l'extrémité proximale du piston s'engage de manière amovible avec l'au moins un cran d'arrêt (36) quand le mécanisme d'entraînement (15, 16, 34) est chargé par le mécanisme de charge.

2. Dispositif de percée (10) selon la revendication 1, dans lequel le mécanisme d'entraînement (15, 16, 34) comprend un ressort d'entraînement (15) qui sollicite dans une direction opposée à la course de percée.

3. Dispositif de percée (10) selon la revendication 1, dans lequel le piston (34) est fixé en translation entre l'extrémité distale du support de lancette et l'extrémité proximale du support de lancette.

4. Dispositif de percée (10) selon la revendication 1, dans lequel le support (20) de lancette est configuré pour se désengager de l'au moins un cran d'arrêt (36) et du mécanisme d'entraînement (15, 16, 34) durant la course de lancée.

5. Dispositif de percée (10) selon la revendication 4, dans lequel le support (20) de lancette comprend une bride surélevée (33) configurée pour se désengager de l'au moins un cran d'arrêt (36) et du mécanisme d'entraînement (15, 16, 34).

6. Dispositif de percée (10) selon la revendication 1, dans lequel le mécanisme de charge comprend un plongeur (50), le plongeur (50) étant supporté en translation par rapport au support (20) de lancette.

7. Dispositif de percée (10) selon la revendication 1, comprenant en outre un logement (12) comprenant une extrémité proximale (13) et une extrémité distale (11) et un coeur creux, l'extrémité proximale (13) comprenant une ouverture ;
dans lequel le support (20) de lancette est supporté en translation dans le logement (12) ;
dans lequel le mécanisme d'entraînement (15, 16, 34) est supporté par rapport au support (20) de lancette, le mécanisme d'entraînement (15, 16, 34) comprenant un état relâché et un état chargé ; et
dans lequel le mécanisme de charge est inséré en translation à travers l'ouverture d'extrémité proximale du logement.

8. Dispositif de percée (10) selon la revendication 7, dans lequel le bras en porte-à-faux s'étend depuis le logement (12).

9. Dispositif de percée (10) selon la revendication 8, dans lequel le support (20) de lancette comprend un corps creux (32) s'étendant depuis l'extrémité proximale du support de lancette vers l'extrémité distale du support de lancette, le corps creux (32) du support de lancette est configuré pour désengager la surface inclinée du mécanisme d'entraînement (15, 16, 34).

10. Dispositif de percée selon la revendication 7, dans lequel le mécanisme d'entraînement comprend un ressort d'entraînement et le piston est supporté en translation entre l'extrémité proximale du support de lancette et l'extrémité distale du support de lancette, dans lequel le piston engage le ressort d'entraînement.

11. Dispositif de percée (10) selon la revendication 10, dans lequel le ressort d'entraînement (15) est comprimé durant l'état chargé du mécanisme d'entraînement.

12. Dispositif de percée (10) selon la revendication 11, dans lequel le ressort d'entraînement (15) est sollicité à l'opposé de la direction d'engagement par poussée du mécanisme d'entraînement.

13. Procédé de charge d'un mécanisme d'entraînement (15, 16, 34) dans un dispositif de percée (10) selon la revendication 1, le procédé comprenant les étapes consistant à :
engager le mécanisme de charge par rapport au mécanisme d'entraînement (15, 16, 34) qui est supporté en translation dans le support (20) de lancette, le mécanisme d'entraînement (15, 16, 34) comprenant un état relâché et un état chargé ;
pousser le mécanisme de charge vers l'extrémité distale du support de lancette ; et
engager de manière amovible le mécanisme d'entraînement (15, 16, 34) par rapport à l'au moins un cran d'arrêt (36) sur le dispositif de percée lorsque le mécanisme d'entraînement (15, 16, 34) est dans l'état chargé.
